# EUROPEAN PATENT APPLICATION

(11) **EP 1 184 501 A2**
(43) Date of publication of application: **06.03.2002**
(21) Application number: 01307102.2
(22) Date of filing: 21.08.2001
(51) Int. Cl.: D04H 13/00

(54) **Composite sheet**

(30) Priority: 28.08.2000 JP 2000257640; 29.09.2000 JP 2000298973
(71) Applicant: UNI-CHARM CORPORATION, Kawanoe-shi Ehime-ken (JP)
(72) Inventor: Taniguchi, Hiroaki, c/o Technical Center, Mitoyo-gun, Kagawa-ken, 769-1602 (JP)
(74) Representative: Parry, Christopher Stephen

(57) **Abstract**

A composite sheet includes a film 2 and a fibrous sheet made of thermoplastic, respectively. The film 2 is formed on its surface opposed to the fibrous sheet 3 with a plurality of bulgy zones extending in one direction in parallel to and spaced apart from one another and substantially flat zones defined between each pair of the adjacent bulgy zone. The film 2 is welded along its bulgy zones to the fibrous sheet 3.

## Description

This invention relates to a composite sheet comprising a thermoplastic synthetic resin film and a thermoplastic synthetic resin fibrous sheet bonded to the film.

Japanese Patent Application Publication No. 1996-126663A describes an absorbent wearing article comprising a liquid-pervious topsheet, a liquid-impervious backsheet and a liquid-absorbent core disposed between these two sheets. The backsheet comprises a non-porous moisture-pervious film made of block copolymerized polyester resin and a fibrous nonwoven fabric bonded to the moisture-pervious film. In this absorbent wearing article, the nonwoven fabric undulates in a transverse direction of the article to form transversely successive gathers in the transverse direction and the moisture-pervious film is bonded to troughs of the nonwoven fabric by means of hot melt adhesive.

In the case of the article disclosed in the Publication, the moisture-pervious film and the nonwoven fabric are bonded together by means of hot melt adhesive and a peel strength between these film and nonwoven fabric may be deteriorated as the adhesive is deteriorated. Depending on an amount of the adhesive used of this purpose, an excessive amount of the adhesive may spread over the film surface prior to its settings and, as a result, may adversely affect the moisture-permeability of the film.

It is an object of this invention to provide a composite sheet comprising a thermoplastic synthetic resin film and a thermoplastic synthetic resin fibrous sheet bonded together so as to be not readily peeled off from each other. It is another object of this invention to provide such composite sheet free from an anxiety that the desired moisture-permeability of the film might be deteriorated.

According to this invention, there is provided a composite sheet comprising a thermoplastic synthetic resin film and a thermoplastic synthetic resin fibrous sheet bonded to at least one of upper and lower surface of the film, the composite sheet.

The improvement according to this invention is in that the film is formed on its surface opposed to the fibrous sheet with a plurality of bulgy zones extending in one direction in parallel and spaced apart from one another and substantially flat zones each defined between each pair of the adjacent bulgy zones wherein the film is welded along the bulgy zones to the fibrous sheet.

This invention may be implemented also in manners as follow:
(1) The film is made of thermoplastic elastomer resin.
(2) The thermoplastic elastomer resin is selected from a group including urethane-, ester- and amide-based thermoplastic elastomer resin and the film is substantially non-porous and moisture-pervious.
(3) The fibrous sheet is formed with a fibrous nonwoven fabric made of the thermoplastic synthetic resin fibers.
(4) The nonwoven fabric is an elastically stretchable nonwoven fabric obtained by melt-spinning thermoplastic elastomer resin and the film is bonded along its bulgy zones to the elastically stretchable nonwoven fabric under no tension.
(5) Each of the bulgy zones has a width of 0.2 ∼ 2.0 mm and the maximum thickness of 40 ∼ 150 µm and each of the flat zones has a thickness of 5 ∼ 100 µm.
(6) The composite sheet has a water-resistance of 49 hpa or higher as measured in accordance with JIS L 1092A method.
(7) The composite sheet has a moisture-permeability of 3000 g/m² · 24 Hr or higher as measured in accordance with JIS L 1099A method.

Fig. 1 is a perspective view showing one embodiment of a composite sheet according to this invention as partially broken away;
Fig. 2 is a sectional view taken along a line A - A in Fig. 1;
Fig. 3 is a sectional view showing a part of Fig. 2 in an enlarged scale;
Fig. 4 is a view similar to Fig. 1 but showing an alternative embodiment of the composite sheet according to this invention; and
Fig. 5 is a sectional view taken along a line B - B in Fig. 4.

Details of a composite sheet according to this invention will be more fully understood from the description given hereunder with reference to the accompanying drawings.

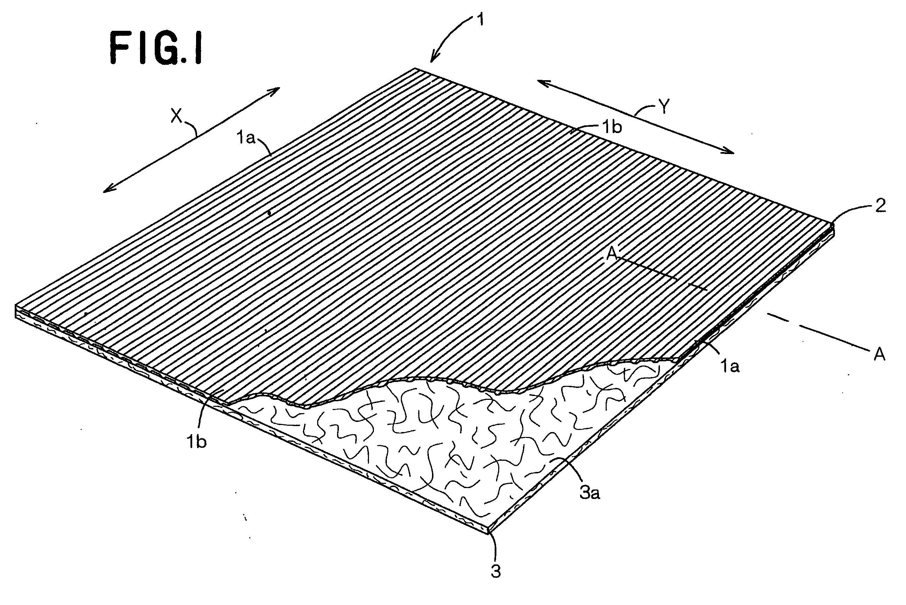

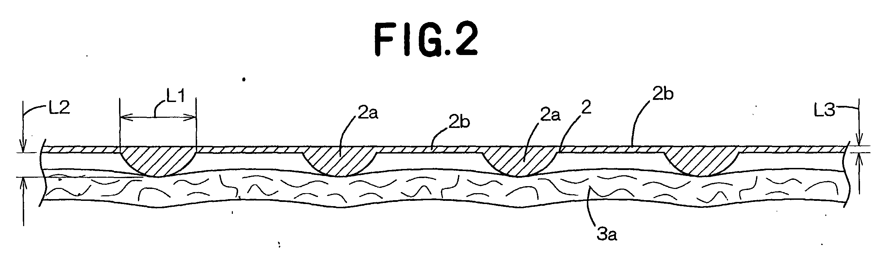

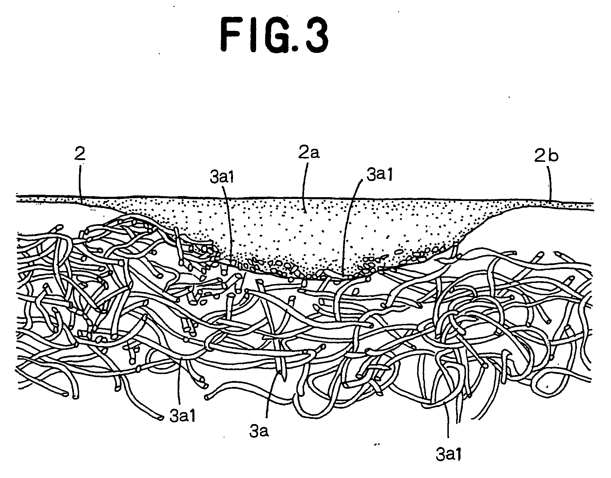

Fig. 1 is a perspective view showing one embodiment of a composite sheet according to this invention as partially broken away, Fig. 2 a sectional view taken along a line A - A in Fig. 1 and Fig. 3 is a sectional view showing a part of Fig. 2 in an enlarged scale. Referring to Fig. 1, a longitudinal direction is indicated by an arrow X and a transverse direction is indicated by an arrow Y.

A composite sheet 1 comprises a thermoplastic synthetic resin film 2 and a thermoplastic synthetic resin fibrous sheet 3. The composite sheet 1 is of a two-layered structure comprising the film 2 and the fibrous sheet 3 placed upon the lower surface of the film 2 and has transversely opposite side edges 1a extending in parallel to each other in the longitudinal direction and longitudinally opposite ends 1b extending in parallel to each other in the transverse direction.

The film 2 is made of ester-based thermoplastic elastomer resin and substantially non-porous and moisture-pervious. The elastomer resin is a block copolymer having comprises hard segments and soft segments. The hard segments are polyester of aromatic dicarboxylic acid and aliphatic diol or of aliphatic dicarboxylic acid and aromatic diol. The soft segments are polyether.

The moisture-permeability of the film 2 is based on a mechanism as will be described. The film 2 comprises the hard segments functioning as molecule constraining components to prevent the film 2 from being plastically deformed and the soft segments as a flexible components having the moisture-permeability. The hard segments and the soft segments are irregularly present in the film 2. In the hard segment, polymer molecular chains of polyester form a crystalline phase. In the soft segment, polymer molecular chains of polyether form an amorphous phase. In the soft segment which is normally at a temperature higher than the glass transition temperature, tight linking of polymer molecular chains of polyether is loosened by microbrownian movement to provide interstices each having a size larger than a molecule of water vapor (particle's diameter of approximately 3.5 Å). The molecules of water vapor are adsorbed on the top surface of the film 2 under an intermolecular force generated in polyester or polyether and then permeate the film 2 through the interstices generated in the soft segments. So far as a vapor pressure on the bottom surface of the film 2 is lower than a vapor pressure on the top surface of the film 2, the molecules of water vapor are forced to move toward the bottom surface of the film 2 through the interstices until they are forced to completely permeate the film 2 and discharged from the bottom surface of the film 2 into the exterior.

The film 2 is formed on its bottom surface with a plurality of bulgy zones 2a and substantially flat zones 2b each defined between each pair of the adjacent bulgy zones 2a. On the film 2, the bulgy zones 2a extend in parallel one to another and are spaced one from another by a predetermined dimension in the transverse direction and the flat zones 2b extend in parallel one to another in the longitudinal direction between respective pairs of the adjacent bulgy zones 2a.

The fibrous sheet 3 is made of an elastically stretchable nonwoven fabric 3a obtained by melt spinning thermoplastic elastomer resin. In the nonwoven fabric 3a, component fibers 3a₁ thereof are intertwined and welded together at crossing points of these component fibers 3a₁.

As will be apparent from Fig. 3, in the composite sheet 1, the film 2 is welded along its bulgy zones 2a to the synthetic resin fibers 3a₁ forming the nonwoven fabric 3a with the synthetic resin fibers 3a₁ being held in a fibrous assembly and thereby the film 2 is integrated with the nonwoven fabric 3a. This composite sheet 1 has a moisture-permeability of 3000 g/m² · 24 Hr or higher and a water-resistance of 49 hpa or higher. The moisture-permeability was measured in accordance with prescription of The Japanese Industrial Standards (hereinafter referred to as "JIS") L 1099A-2 method and the water-resistance was measured in accordance with prescription of JIS L 1092A method. The composite sheet 1 presents a high moisture-permeability and simultaneously presents a high liquid-impermeability.

In the composite sheet 1, a peel strength of 400 mN/25 mm or higher is preferably established between the film 2 and the nonwoven fabric 3a. If the peel strength is lower than 400 mN/25 mm, the film 2 and the nonwoven fabric 3a will be readily separated from each other and it will be impossible to maintain a desired shape for the composite sheet 1.

In the film 2, each of the bulgy zones 2a has a width L1 of 0.2 ∼ 2.0 mm and the maximum thickness L2 of 40 ∼ 150 µ m. With the width L1 less than 0.2 mm and the maximum thickness L2 of the bulgy zone 2a less than 40 µm, the peel strength between the nonwoven fabric 3a and the film 2 will be deteriorated and it will be difficult to ensure the peel strength of 400 mN/25 mm or higher between the nonwoven fabric 3a and the film 2. With the width L1 exceeding 2.0 mm and the maximum thickness L2 of the bulgy zone 2a exceeding 150 µm, a stiffness of the film 2 will unacceptably increase and a desired flexibility of the composite sheet 1 will be deteriorated.

In the film 2, each of the flat zones 2b has a thickness L3 of 5 ∼ 100 µm. With the thickness L3 of the flat zone 2b less than 5 µm, a strength of the film 2 at the flat zones 2b will be deteriorated and may result in breakage of the film 2. With the thickness L3 of the flat zone 2b exceeding 100 µm, the moisture-permeability of the film 2 in the flat zones 2b will be deteriorated and it will be difficult for the composite sheet 1 to ensure a moisture-permeability of 3000 g/m²/day.

The composite sheet 1 has an elastic stretchability and a high peel strength so that the composite sheet 1 can be stretchable in length and width and no separation occurs between the film 2 and the nonwoven fabric 3a even when the composite sheet 1 is stretched.

In the composite sheet 1, the film 2 may be formed with any one of urethane-based thermoplastic elastomer resin and amide-based thermoplastic elastomer resin. Alternatively, the film 2 may be formed with any one of following types of resin: styrene-based thermoplastic elastomer resin, 1, 2-polybutadiene-based thermoplastic elastomer resin, olefine-based thermoplastic elastomer resin, chlorinated polyethylene-based thermoplastic elastomer resin, polyvinyl chloride-based thermoplastic elastomer resin and polyfluorocarbon-based thermoplastic elastomer resin.

In the composite sheet 1, the nonwoven fabric 3a may be of an inelastically stretchable nonwoven fabric made of polyolefine-based fiber such as polypropylene or polyethylene fiber; polyester-base fiber such as polyethylene terephthalate or polybutylene terephthalate; polyamide-based fiber such as nylon 66 or nylon 6 fiber; acryl-based fiber; core-sheath type conjugated fiber; or side-by-side type conjugated fiber. Such nonwoven fabric 3a may be obtained by any one of spun lace-, needle punch-, melt blown-, thermal bond-, spun bond-, chemical bond- and air through-processes. Alternatively, it is also possible to form the nonwoven fabric 3a with a composite nonwoven fabric comprising a melt blown nonwoven fabric having a high water-resistance sandwiched by two layers of spun bond nonwoven fabric each having a high strength and a high flexibility.

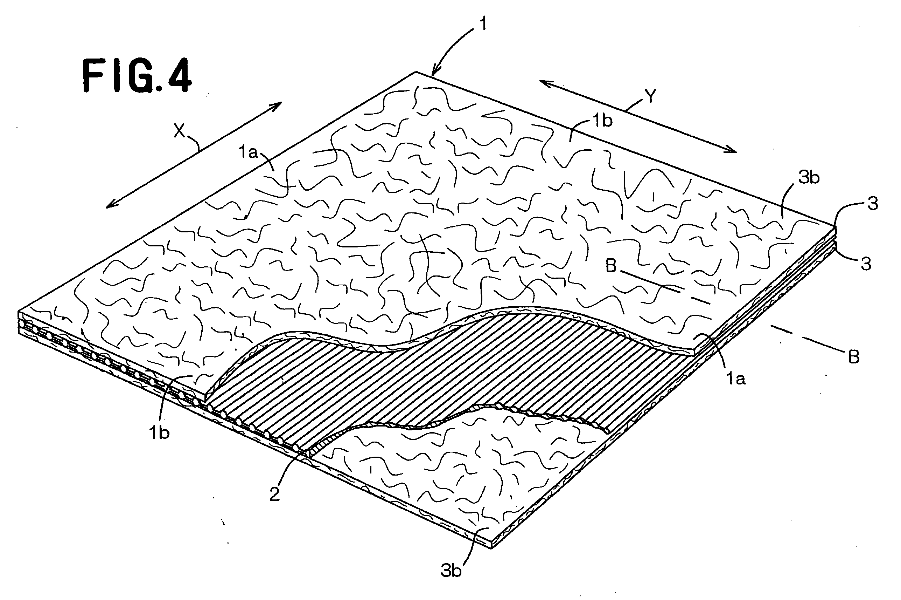

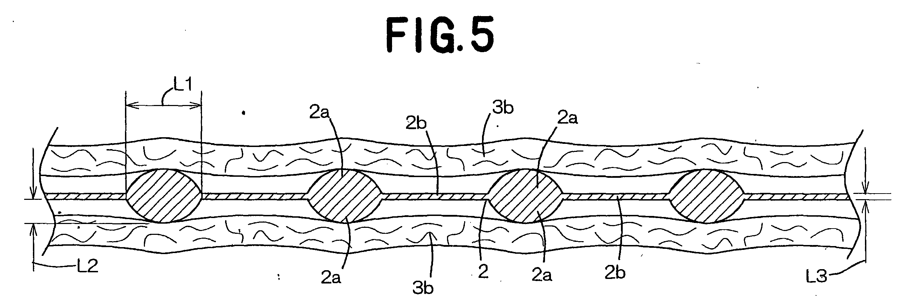

Fig. 4 a view similar to Fig. 1 but showing an alternative embodiment of the composite sheet according to this invention and Fig. 5 a sectional view taken along a line B - B in Fig. 4. Referring to Fig. 4, the longitudinal direction is indicated by the arrow X and the transverse direction is indicated by the arrow Y. The composite sheet 1 of Fig. 4 is different from the composite sheet 1 of Fig. 1 in that the composite sheet 1 is three-layered, i.e., comprises the plastic film 2 and two layers of sheet member 3 placed upon the upper and lower surfaces of the plastic film 2, respectively.

The film 2 is formed on its upper and lower surfaces with a plurality of bulgy zones 2a and substantially flat zones 2b defined between each pair of the adjacent bulgy zones 2a. The bulgy zones 2a extending in parallel one to another in the longitudinal direction and spaced one from another by a predetermined dimension in the transverse direction. The flat zones 2b extend in parallel one to another in the longitudinal direction between respective pairs of the adjacent bulgy zones 2a.

Similarly to the film 2 of Fig. 1, the film 2 is formed with ester-based thermoplastic elastomer resin and substantially non-porous but moisture-permeable. The fibrous sheet 3 comprises fibrous nonwoven fabric 3b formed with thermoplastic synthetic resin. The nonwoven fabric 3b is preferably stretchable one obtained by spun lace- or spun bond process.

In the composite sheet 1, the film 2 is welded along its bulgy zones 2a to the synthetic resin fibers forming the nonwoven fabric 3b with the synthetic resin fibers being held in a fibrous assembly and thereby the film 2 is integrated with the nonwoven fabric 3b. In the composite sheet 1 according to this embodiment shown by Fig. 4, the width L1 as well as the maximum thickness L2 of the bulgy zone 2a and the thickness L3 of the flat zone 2b are same as those in the embodiment shown by Fig. 1.

These composite sheets 1 shown in Figs. 1 and 4 are suitable as the component members in a sanitary wearing article such as a disposable diaper, a sanitary napkin, an urine-absorbent pad for incontinent patient, a diaper cover or sanitary shorts.

The stock material for the sheet member 3 is not limited to the nonwoven fabric 3a, 3b and it is also possible to use woven or knitted fabric made of thermoplastic synthetic resin fiber such as polyolefine-, polyester-, polyamide- or acryl-based fiber as the stock material for the sheet member 3. The woven or knitted fabric preferably comprises elastic yarn such as false twisted yarn, covered yarn, core spun yarn, plied yarn or air covered yarn. The composite sheet 1 using woven or knitted fabric made of false twisted yarn or elastic yarn as the sheet member 3 is useful as the stock material for comfort stretch clothes such as working clothes, slacks, a jacket or suits; performance stretch clothes such as a training wear or a baseball uniform; or power stretch clothes such as a leotard or spats. The composite sheet 1 is applicable also to the other article such as a tent cloth, gloves, a hat or socks.

The composite sheet according to this invention comprises the thermoplastic synthetic resin film is welded along its bulgy zones to the synthetic resin fibers forming the fibrous sheet so that the film is integrated with the fibrous sheet and has a high peel strength. The film and the fibrous sheet are not adhesively bonded but welded to each other and there is no anxiety that the peel strength might be deteriorated as adhesive agent is deteriorated.

The composite sheet comprising the film made of thermoplastic elastomer resin and the fibrous sheet made of elastically stretchable nonwoven fabric not only presents a high peel strength but also an elastic stretchability as well as high water-resistance. The composite sheet in which the thermoplastic elastomer resin is any one of urethane-, ester- and amide-based resin and the fibrous sheet is formed with elastically stretchable nonwoven fabric presents, in addition to a high peel strength, an elastic stretchability, a high water-resistance and a high moisture-permeability. The composite sheet having an elastic stretchability is less likely that the film and the nonwoven fabric might be separated from each other even when the composite sheet is stretched.

## Claims

1. A composite sheet comprising a thermoplastic synthetic resin film and a thermoplastic synthetic resin fibrous sheet bonded to at least one of upper and lower surfaces of said film, wherein:
said film is formed on its surface opposed to said fibrous sheet with a plurality of bulgy zones extending in one direction in parallel and spaced apart from one another and substantially flat zones each defined between each pair of the adjacent bulgy zones wherein said film is welded along said bulgy zones to said fibrous sheet.

2. The composite sheet according to Claim 1, wherein said film is made of thermoplastic elastomer resin.

3. The composite sheet according to Claim 2, wherein said thermoplastic elastomer resin is selected from a group including urethane-, ester- and amide-based thermoplastic elastomer resin and said film is substantially non-porous and moisture-pervious.

4. The composite sheet according to Claim 1, wherein said fibrous sheet is formed with a fibrous nonwoven fabric made of said thermoplastic synthetic resin fibers.

5. The composite sheet according to Claim 4, wherein said nonwoven fabric is an elastically stretchable nonwoven fabric obtained by melt-spinning thermoplastic elastomer resin and said film is bonded along its bulgy zones to said elastically stretchable nonwoven fabric under no tension.

6. The composite sheet according to Claim 1, wherein each of said bulgy zones has a width of 0.2 ∼ 2.0 mm and the maximum thickness of 40 ∼ 150 µm and each of said flat zones has a thickness of 5 ∼ 100 µm.

7. The composite sheet according to Claim 1, wherein said composite sheet has a water-resistance of 49 hpa or higher as measured in accordance with JIS L 1092A method.

8. The composite sheet according to Claim 1, wherein said composite sheet has a moisture-permeability of 3000 g/m²·24 Hr or higher as measured in accordance with JIS L 1099A method.
